# EUROPÄISCHE PATENTSCHRIFT

(11) **EP 0 629 600 B1**
(45) Veröffentlichungstag und Bekanntmachung des Hinweises auf die Patenterteilung: **07.10.1998**
(21) Anmeldenummer: 94108437.8
(22) Anmeldetag: 01.06.1994
(51) Int. Cl.: C07C 29/76, C07C 68/08, C07C 41/58, B01D 61/36, B01D 69/12, B01D 71/38

(54) **Verfahren zur Abtrennung von Alkanolen von weiteren organischen Verbindungen mit höherer Kohlenstoffzahl**
Process for the separation of alkanols from further organic compounds with a higher number of carbon atoms
Procédé pour la séparation d'alcanols d'autres composés organiques avec un nombre plus élevé d'atomes de carbone

(30) Priorität: 14.06.1993 DE 4319570
(43) Veröffentlichungstag der Anmeldung: 21.12.1994
(73) Patentinhaber: BAYER AG, 51368 Leverkusen (DE)
(72) Erfinder: Mehl, Wolf, Dr., D-50733 Köln (DE); Scheinert, Wolfgang, Dr., D-51375 Leverkusen (DE); Janisch, Ingo, Dr., D-51515 Kürten (DE); Gröschl, Andreas, Dr., D-51375 Leverkusen (DE)

(56) Entgegenhaltungen:
- EP-A- 0 331 846
- EP-A- 0 423 949
- EP-A- 0 592 883

## Beschreibung

Die vorliegende Erfindung betrifft ein Verfahren zur Abtrennung niederer Alkanole von weiteren organischen Verbindungen aus der Gruppe von weiteren Alkoholen, Polyalkoholen, Ethern, Oxoverbindungen, Estern von Carbonsäuren und der Kohlensäure, Halogen-Aliphaten, Aminen, Amiden, Kohlenwasserstoffen, Carbonsäuren, Nitrilen oder Gemischen mehrerer von ihnen. Die weiteren organischen Verbindungen haben stets mindestens 1 C-Atom mehr als das abzutrennende Alkanol, wobei im Falle von Halogen-Aliphaten Halogensubstituenten als weitere C-Atome gezählt werden. Die Abtrennung erfolgt durch Pervaporation oder Dampfpermeation an einer hydrophilen Membran. Erfindungsgemäß wird hierzu ein Gehalt an Wasser von 1 bis 30 Gew.-%, bevorzugt 5 bis 15 Gew.-%, bezogen auf die Menge von Gemisch und Wasser, aufrechterhalten bzw. eingestellt und aufrechterhalten.

Das erfindungsgemäße Verfahren ermöglicht vereinfachte Trennungen, insbesondere in solchen Fällen, in denen durch Vorliegen eines Azeotrops destillative Trennverfahren versagen. Aber auch bei anderen Trennproblemen ergeben sich durch das erfindungsgemäße Verfahren wesentliche Vorteile im Energieaufwand. Wichtige technische Probleme dieser Art sind beispielsweise die Abtrennung von Methanol aus i-Propanol, Dimethylcarbonat (DMC), Methyl-tert.-butylether (MTBE) und tert.-Amyl-methyl-ether (TAME). Die Trennung solcher Gemische erfolgte bisher beispielsweise im Falle von DMC durch Zweidruckdestillation (DE-A 26 07 003), da es bekannt ist, daß die Zusammensetzung von azeotropen Gemischen druckabhängig ist. Vielfach reicht die hierbei erhaltene Trennrate nicht aus und muß ergänzt werden durch andere physikalische Prozesse, beispielsweise durch eine Kristallisation. Zudem verursachen Druckapparaturen stets höhere Investitionskosten; wegen des durch den Druck erhöhten Temperaturniveaus ist stets auch mit einer erhöhten Bildung von Nebenprodukten zu rechnen. Neben der Destillation unter erhöhtem Druck wurde auch versucht, Gemische der beschriebenen Art durch eine Extraktivdestillation zu trennen. Im Falle der Auftrennung des Azeotrops Methanol/DMC wird hierbei bevorzugt Wasser als Extraktionsmittel verwendet (DE-A 24 50 856). Das benötigte Verhältnis von Wasser zu Dimethylcarbonat liegt dabei bei 20. Diese große Wassermenge muß in einer weiteren Destillationskolonne wieder vom Methanol getrennt werden. Hierbei ist es zusätzlich nachteilig, daß Wasser im Gegensatz zu organischen Verbindungen eine mehr als 4 mal so hohe Verdampfungswärme und eine mehr als doppelt so hohe Warmekapazität hat; beides führt zu einem erhöhten Energieverbrauch. Auch eine Verbesserung der Extraktivdestillation durch Verwendung von organischen Lösungsmitteln an Stelle von Wasser ist nach wie vor nachteilig durch die Notwendigkeit, diese Zusatzstoffe aufzuarbeiten und zu recyclisieren (EP-A 1780; DE-A 27 06 684 und DE-A 27 37 265).

Es ist daher bereits versucht worden, schwierig zu trennende Gemische mit Hilfe der Membrantechnologie aufzutrennen. So beschreibt EP-A 331 846 die Trennung von kurzkettigen Alkoholen von sauerstoffhaltigen organischen Verbindungen, wie Ethern, Aldehyden, Ketonen oder Estern, mit Hilfe einer Mehrschichtmembran. Die Trennmembran dieser Anordnung besteht entweder aus einer Polyvinylalkohol-Membran, die mit aliphatischen Polyaldehyden vernetzt ist, oder aus einem Harz, das auch bei Ionenaustauschern eingesetzt wird und Säuregruppen enthält, die mit quarternären Ammoniumsalzen modifiziert wurden. Als Trägermaterial für die Mehrschichtmembran wird ein Polyestergewebe verwendet; eine weiterhin benutzte poröse Stützmembran besteht aus einem Polysulfongewebe. Diese Membran ist relativ kompliziert aufgebaut. Infolge ihres chemischen Aufbaus ist die Arbeitstemperatur auf 70°C begrenzt und ergibt hierbei einen niedrigen maximalen Fluß von 1,5 kg/m².h. Dies setzt dem technischen Einsatz Grenzen. Die beschriebenen Permeatanreicherungen an Methanol von 73 % auf lediglich 93 % im Falle der Trennung von Methanol/DMC bedeuten, daß das hierbei gewonnene Methanol in einem weiteren Arbeitsgang von den restlichen 7 % an DMC befreit werden muß.

Gemäß Beschreibung in EP-A 423 949 wird versucht, die beschriebenen Nachteile des Verfahrens von EP-A 331 846 durch eine andere Membran zu überwinden, die ein Blend aus Polyvinylalkohol und Polyacrylsäure auf Polyacrylnitril als Stützmembran darstellt. Im Falle der Trennung von Methanol/DMC wird eine Anreicherung von 73 % Methanol auf etwa 95 % Methanol bei einem Fluß von etwa 2 kg/m².h erreicht; diese Anreicherung stellt auf der Retentatseite die Mindestanforderung für das gereinigte DMC zur Verwendung in weiteren Prozessen dar. Eine durch Plasmapolymerisation erhaltene Membran, die bevorzugt eine Composit-Membran darstellt, wird gemäß Beschreibung von EP-A 476 370 zur Abtrennung von Reaktionswasser aus einem Veresterungsgemisch eingesetzt. Ein Veresterungsgemisch gemaß dieser Beschreibung besteht aus nicht umgesetzter Carbonsäure, nicht umgesetztem Alkohol, dem als Reaktionsprodukt gewünschten Ester, dem dabei entstandenen Reaktionswasser und einem sauren Veresterungskatalysator. Der Abtrennung des Reaktionswassers an der Membran folgt sodann eine Auftrennung des Retentats in den Ester-Produktstrom und in die zurückzuführenden, noch nicht umgesetzten Säuren und Alkohole. Das Verfahren von EP-A 476 370 ist demnach dadurch gekennzeichnet, daß außer dem Wasser keine wesentlichen organischen Bestandteile durch die Membran treten; insbesondere wichtig ist hierbei, daß der Veresterungsalkohol nicht durch die Membran tritt.

Es wurde nun gefunden, daß es besonders günstig ist, C₁-C₃-Alkanole aus ihren Gemischen mit weiteren organischen Verbindungen, die mindestens 1 C-Atom mehr haben als dieses erste Alkanol oder bei gleicher C-Anzahl zusätzlich Halogenatome enthalten, abzutrennen, wenn man zusätzlich zum Gemisch aus dem niederen Alkanol und der weiteren organischen Verbindung einen weiter unten naher beschriebenen Gehalt an Wasser innerhalb dieses Gemisches aufrechterhält bzw. einstellt und die Trennung an einer hydrophilen Membran vornimmt. Es ist möglich, daß zu Beginn der Abtrennung des niederen Alkanols der Gehalt an Wasser durch ein im Gemisch ursprünglich vorhandenen Anteil Wasser repräsentiert wird, der nach seinem Verbrauch durch Zugabe von weiterem Wasser so ergänzt wird, daß der erfindungsgemäße Gehalt an Wasser aufrechterhalten wird, bis das niedere Alkanol auf eine vorher bestimmte Konzentration gebracht worden ist. Nach der Abtrennung des C₁-C₃-Alkanols wird das hierzu benötigte Wasser ebenfalls abgetrennt. Das Permeat aus dem niederen Alkanol und Wasser kann dann durch bekannte Maßnahmen aufgearbeitet werden.

Die Erfindung betrifft demnach ein Verfahren zur Abtrennung eines ersten Alkanols mit 1 bis 3 C-Atomen aus ihrem Gemisch mit weiteren organischen Verbindungen aus der Gruppe, die ein zweites geradkettiges oder verzweigtes, offenkettiges oder cyclisches gesättigtes oder ungesättigtes C₂-C₁₀-Alkanol, einen C₂-C₈-Polyalkohol, einen geradkettigen oder verzweigten, offenkettigen oder cyclischen C₄-C₈-Ether oder -Polyether, eine geradkettige oder verzweigte, cyclische oder offenkettige C₂-C₆-Oxoverbindung, einen geradkettigen, cyclischen oder verzweigten C₂-C₉-Carbonsäureester, einen C₃-C₉-Kohlensäureester, einen Phosphorsäureester mit C₁-C₄-Alkylgruppen, einen 5 bis 7-gliedrigen aromatischen oder nicht aromatischen N-Heterocyclus, ein Sulfoxid oder Sulfon mit 2 bis 8 C-Atomen, einen C₁-C₄-Halogenaliphaten, ein C₃-C₈-Amin, ein C₃-C₈-Amid, einen C₅-C₈-Kohlenwasserstoff, eine geradkettige oder verzweigte C₂-C₆-Carbonsäure und ein C₂-C₆-Nitril umfaßt, wobei das erste Alkanol stets mindestens 1 C-Atom, vorzugsweise 2 C-Atome weniger hat als jede der weiteren organischen Verbindungen, wobei im Falle von Halogen-Aliphaten Halogensubstituenten als weitere C-Atome zählen, durch Pervaporation oder Dampfpermeation, das dadurch gekennzeichnet ist, daß das beschriebene Gemisch unter Aufrechterhaltung eines Gehaltes an H₂O von 1 bis 30 Gew.-%, bevorzugt 5 bis 15 Gew.-%, bezogen auf die Menge von Gemisch und H₂O, bei 40 bis 130°C, bevorzugt bei 40 bis 100°C einer hydrophilen Membran zugeführt wird, wobei auf der Feed-Seite ein Druck von 0,5 bis 10 bar, bevorzugt 0,8 bis 6 bar, besonders bevorzugt 1 bis 5 bar, und auf der Permeatseite ein Druck von höchstens 100 mbar, bevorzugt höchstens 20 mbar, eingestellt wird und wobei als Permeat das erste Alkanol im Gemisch mit H₂O und als Retentat die weitere organische Verbindung in angereicherter Form erhalten werden und wobei nach Abtrennung des ersten Alkanols das vorhandene Wasser ebenfalls abgetrennt wird.

Die Abtrennung des C₁-C₃-Alkanols im erfindungsgemäßen Sinne kann von einer Reihe chemisch untereinander verschiedenartiger Verbindungen erfolgen, die alle eine Mischbarkeit mit solchen Alkanolen aufweisen. Solche Verbindungen sind:
- C₂-C₁₀-Alkanole, die geradkettig oder verzweigt, offenkettig oder cyclisch, gesättigt oder ungesättigt sein können, bevorzugt C₃-C₆-Alkanole;
- C₂-C₈-Polyalkohole, bevorzugt C₂-C₄-Polyalkohole;
- C₄-C₈-Ether, die geradkettig oder verzweigt, offenkettig oder cyclisch sein können, bevorzugt C₄-C₆-Ether;
- C₂-C₆-Oxoverbindungen, die geradkettig oder verzweigt, cyclisch oder offenkettig sein können und Aldehyde und Ketone umfassen;
- C₂-C₉-Carbonsäureester, die geradkettig oder verzweigt sein können, bevorzugt C₃-C₆-Carbonsäureester;
- C₃-C₉-Kohlensäureester, bevorzugt C₃-C₅-Kohlensäureester, besonders bevorzugt symmetrische Kohlensäureester;
- Phosphorsäureester mit C₁-C₄-Alkylgruppen;
- C₁-C₄-Halogen-Aliphaten;
- C₃-C₈-Amine;
- C₃-C₈-Amide;
- C₅-C₈-Kohlenwasserstoffe, die geradkettig oder verzweigt, offenkettig oder cyclisch sein können;
- C₂-C₆-Carbonsäuren, die geradkettig oder verzweigt sein können; bevorzugt C₂-C₄-Carbonsäuren;
- C₂-C₆-Nitrile;
- 5- bis 7-gliedrige aromatische und nicht aromatische N-Heterocyclen;
- Sulfoxide und Sulfone mit 2 bis 8 C-Atomen.

Beispiele für solche weiteren organischen Verbindungen sind: Ethanol, n-Propanol, i-Propanol, n-Butanol, i-Butanol, tert.-Butanol, n-Amylalkohol, 3-Methyl-butanol-1 (i-Amylalkohol), 2-Methyl-butanol-1, Pentanol-2, tert.-Amylalkohol, Allylalkohol, Cyclohexanol, Furfurylalkohol, Hexanol, Glykol, Glykolmonomethylether, Glykoldimethylether, Propandiol-1,2, Propandiol-1,3, Butandiol-1,4, Hexandiol-1,6, Glycerin, Diethylenglykol, Diethylenglykolmonomethylether, Tetraethylenglykol, Butantriol, Diethylether, Diisopropylether, Dipropylether, Methyl-tert.-butylether (MTBE), tert.-Amyl-methylether (TAME), Ethyl-propylether, Allylether, Dioxan-1,4, Tetrahydrofuran, Furfurol, Methyl-furfurol, Acetaldehyd, Propionaldehyd, Aceton, Butanon, Methyl-ethyl-keton, Cyclohexanon, 2-Methyl-pentanon (Methyl-i-butyl-keton), Pentanon-2, Pentanon-3, 1,3-Dioxolan, Acetonylaceton, Acetylaceton, Dimethylglyoxal, Diaceton-alkohol, Methylacetat, Ethylacetat, n-Amylacetat, n-Butylacetat, i-Butylacetat, i-Propylacetat, i-Amylacetat, Methylformiat, Ethylformiat, Benzylformiat, Butylformiat, Ethylbutyrat, i.-Amylbutyrat, Methyl-butyrat, i-Butylbutyrat, Butylpropionat, i-Butylpropionat, i-Amylpropionat, Chloraceton, Trichlorethylen, Tetrachlorethan, Chloroform, Dichlorpropan, Chlorbenzol, N,N-Dimethylformamid, N,N-Dimethylacetamid, Ethanolamin, Ethylendiamin, tert.-Amylamin, Diethylamin, Dichlorpropan, Isopropylamin, Piperidin, Triethanolamin, Triethylamin, Anilin, Hexylamin, Dibutylamin, Benzol, Toluol, Xylol, Kerosin, Petrolether, Cyclohexan, Essigsäure, Propionsäure, Buttersäure, Capronsäure, Dimethylcarbonat, Methyl-ethyl-carbonat, Diethylcarbonat, Dipropylcarbonat, Dibutylcarbonat, Di(i-butyl)-carbonat, Acetonitril, Propionitril, Butyronitril, Acrylnitril, Diethoxymethan, Morpholin, N-Methyl-pyrrolidon (NMP), N-Methyl-caprolactam (NMC), Dimethylsulfoxid, Sulfolan, Picoline, Pyridine, Phosphorsäuretriester, Pyridone, Cyclopropylcarbonsäureethylester, Cyclopropylcarbonsäureamid, Caprolactam.

Aus der Auflistung ist erkennbar, daß weitere organische Verbindungen, die in einer bestimmten Gruppe aufgelistet sind, auch zusätzliche funktionelle Gruppen tragen können, wie beispielsweise Diaceton-Alkohol, Chloraceton, Ethanolamin, Triethanolamin usw..

Selbstverständlich ergibt es sich in naheliegender Weise, daß ein niederes C₁-C₃-Alkanol auch aus einem Gemisch mehrerer der oben aufgelisteten weiteren organischen Verbindungen abgetrennt werden kann. Solche Gemische sind beispielsweise Ethylacetat/Ethanol, Diethylether/Ethanol, Acetate/Ethanol, Aceton/i-Propylether, Allylalkohol/Allylether, Allylalkohol/Cyclohexan, Benzol/Ethanol, Butanol/Butylacetat, Butanol/Dibutylether, Chloroform/Ethanol, Ethanol/Ethylbutylether, Propylacetat/Propanol, Isopropylether/i-Propanol, Ethanol/i-Propanol, Ethylacetat/Ethanol/Essigsäure und weitere Kombinationen die sich zwanglos aus der obigen Auflistung ergeben.

Als weitere organische Verbindungen, von denen erfindungsgemäß ein C₁-C₃-Alkanol abgetrennt wird, ist bevorzugt eine aus der Gruppe der obengenannten C₂-C₁₀-Alkohole, der C₂-C₈-Polyalkohole, der C₄-C₈-Ether, der C₂-C₆-Oxoverbindungen, der C₂-C₉-Carbonsäureester, der C₃-C₉-Kohlensäureester, der C₃-C₈-Amine, der C₃-C₈-Säureamide und der C₂-C₆-Carbonsäuren. In besonders bevorzugter Weise entstammt die weitere organische Verbindung der Gruppe der C₂-C₁₀-Alkohole, der C₄-C₈-Ether, der C₂-C₆-Oxoverbindungen, der C₂-C₉-Carbonsäureester oder der C₃-C₉-Kohlensäureester.

C₁-C₃-Alkanole, die von weiteren organischen Verbindungen abgetrennt werden, sind Methanol, Ethanol, n-Propanol oder i-Propanol, bevorzugt Methanol oder Ethanol, besonders bevorzugt Methanol. Es kann auch ein Gemisch mehrerer von ihnen abgetrennt werden.

Als besonders wichtige Trennaufgaben der chemischen Technik seien bezeichnet: die Abtrennung von Methanol aus seinem Gemisch mit einer weiteren organischen Verbindung aus der Gruppe von geradkettigen oder verzweigten C₃-C₆-Alkanolen, DMC, MTBE oder TAME.

Das erfindungsgemäße Verfahren wird bei einer Feed-Temperatur von 40 bis 130°C, bevorzugt 40 bis 100°C durchgeführt. Auf der Feed-Seite wird ein Druck von 0,5 bis 10 bar, bevorzugt 0,8 bis 6 bar, besonders bevorzugt 1 bis 5 bar, eingestellt. Auf der Permeatseite wird ein Druck von höchstens 100 mbar, bevorzugt höchstens 20 mbar, beispielsweise 1 bis 100 mbar, bevorzugt 1 bis 20 mbar, eingestellt.

Bei der Durchführung des erfindungsgemäßen Verfahrens als Pervaporation wird das Feed-Gemisch in flüssiger Form an die Membran herangeführt, und der Druck und die Temperatur auf der Feed-Seite werden so eingestellt, daß der Druck beim Siededruck oder oberhalb des Siededruckes des Feed-Gemisches liegt. In einer bevorzugten Ausführungsform wird die Pervaporation so durchgeführt, daß das Feed-Gemisch der Membran in siedender Form zuläuft.

Es ist jedoch ebenfalls möglich, das erfindungsgemäße Verfahren in Form der Dampfpermeation durchzuführen, wobei das Feed-Gemisch dampfförmig an die Membran gebracht wird. In diesem Falle werden Drücke und Temperaturen innerhalb der angegebenen Bereiche so gewählt und kombiniert, daß der Druck in Abhängigkeit von der Temperatur unterhalb des Dampfdruckes des Feed-Gemischs liegt. Gegebenenfalls kann im Fall der Dampfpermeation das dampfförmige Feed-Gemisch mit Hilfe eines Trägergasstromes an die Membran herangebracht werden, der aus einem inerten Gas oder einer Mischung mehrerer inerter Gase besteht. Brauchbare inerte Gase sind beispielsweise Stickstoff, die Edelgase, niedere Kohlenwasserstoffe, Luft, Kohlenmonoxid oder Kohlendioxid.

Das Permeat fällt zunächst dampfförmig an und kann in dieser Form von der Membran abgeführt oder vorher kondensiert und als Kondensat dem Permeatraum entnommen werden. Für den Fall einer gasförmigen Abführung kann auch hier ein inertes Trägergas der oben beschriebenen Art eingesetzt werden.

Der Aufbau einer Apparatur zur Durchführung des erfindungsgemäßen Verfahrens ist einfach und besteht aus einem Vorratsbehälter für das zu trennende Gemisch, einer Pumpe zur Einstellung des gewünschten Feed-Druckes, einem Modul mit der erfindungsgemäß einzusetzenden hydrophilen Membran, einer Abführung des auf der Anströmseite der Membran zurückbleibenden Retentats und einer Abführungsmöglichkeit für das Permeat, wobei letztere vor allen Dingen durch die zur Aufrechterhaltung des Unterdruckes erforderliche Vakuumpumpe gekennzeichnet ist. Die Permeatseite kann vor oder hinter der Vakuumpumpe einen Kondensator aufweisen. Es ist weiterhin möglich, das erfindungsgemäße Verfahren durch Behandlung einer vorbestimmten Gemischmenge quasi absatzweise zu betreiben oder es in vollkontinuierlicher Weise zu betreiben. Bei der absatzweisen Durchführung ist es weiterhin möglich, das Retentat in den Vorratsbehälter für das zu trennende Gemisch zurückzuführen und so die Gemischmenge wiederholt an die Membran zu führen, bis ein gewünschter Trenneffekt erreicht ist. Bei der vollkontinuierlichen Durchführung ist es weiterhin möglich, das Retentat an ein weiteres Modul zu bringen und somit eine mehrstufige Behandlung bis zur Erreichung des gewünschten Trenneffektes durchzuführen.

Erfindungsgemäß wird dem Feed-Gemisch so viel Wasser zugesetzt, daß ein Gehalt an H₂O von 1 bis 30 Gew.-%, bezogen auf die Menge von Gemisch und H₂O, aufrechterhalten wird. In bevorzugter Weise wird ein Gehalt an H₂O von 5 bis 15 Gew.-% aufrechterhalten. Dieses Wasser wird dem zu trennenden Gemisch aus niederem Alkanol und weiterer organischer Verbindung entweder vor dessen Eintritt in das Membranmodul zugemischt oder über eine separate Leitung zeitgleich der Anströmseite der Membran zugeführt. Es ist weiterhin möglich und entspricht der Realität der chemischen Technik, daß die zu trennenden Gemische bereits einen Gehalt an Wasser aufweisen. Dieser mit dem zu trennenden Gemisch ursprünglich in die Reaktion eingebrachte Gehalt an Wasser wird jedoch nach seinem Verbrauch im weiteren Verlauf des Verfahrens durch erfindungsgemäß zugesetztes Wasser ergänzt, so daß die obengenannte Menge an Wasser im zu trennenden Gemisch in dem Membranmodul ständig aufrechterhalten wird. Nach Abtrennung des C₁-C₃-Alkanols von der weiteren organischen Verbindung, gegebenenfalls bis hin zu einem vorher bestimmten Maß, wird noch vorhandenes restliches Wasser abgetrennt, so daß die weitere organische Verbindung frei von C₁-C₃-Alkanol und frei von Wasser erhalten wird, bzw. eine vorbestimmte obere Spezifikationsgrenze an diesen Stoffen aufweist.

Das erfindungsgemäße Verfahren wird an einer hydrophilen Membran durchgeführt. Solche Membranen können beispielsweise aus Cellulosediacetat, Cellulosetriacetat oder Polyvinylalkohol hergestellt werden oder eine durch Plasmapolymerisation hergestellte porenfreie Schicht darstellen. Die Polymermaterialien haben hierbei vielfach ein Molekulargewicht zwischen 15.000 und 200.000. Polyvinylalkohol wird im allgemeinen durch weitgehende Verseifung von Polyvinylacetat hergestellt; die Verseifungsgrade sollen bevorzugt über 95 %, besonders bevorzugt über 98 % liegen. Wegen der Wasserlöslichkeit von Polyvinylalkohol wird dies im allgemeinen in vernetzter Form eingesetzt. Solche Vernetzung kann in einer Veretherung, Veresterung oder Acetalisierung mit mehrfunktionellen Verbindungen bestehen. Solche Membranen sind dem Fachmann grundsätzlich bekannt.

In bevorzugter Form werden Composit-Membranen eingesetzt, die im allgemeinen aus mehreren Schichten, nämlich einer Trägerschicht, einer porösen Stützschicht und der eigentlichen Trennschicht bestehen (EP-A 96 339; DE-A 39 39 841). Als Trägerschicht kommen im allgemeinen hochporöse flexible Gewebe oder Vliese aus Fasern, einschließlich Metallfasern, Polyolefinen, Polysulfonen, Polyetherimiden, Polyphenylensulfiden oder Kohlenstoff in Frage; gleichfalls geeignet sind poröse Strukturen aus Glas, Keramik, Graphit oder Metallen. Die poröse Stutzschicht hat bevorzugt eine asymmetrische Porenstruktur. Solche porösen Stutzschichten können beispielsweise aus Polysulfon, Polyethersulfon, Polyetherimid, Polyvinylidenfluorid, hydrolysiertem Cellulosetriacetat, Polyphenylensulfid, Polyacrylnitril, Polytetrafluorethylen, Polyethylen, Polyvinylalkohol, Copolymeren von teilfluorierten Polyolefinen, sowie anderen geeigneten Polymerisaten hergestellt werden. Die Molekulargewichte können gleichfalls im Bereich von 15.000 bis 200.000 liegen. Die eigentliche Trennschicht kann wiederum aus Cellulosediacetat, Cellulosetriacetat oder Polyvinylalkohol bestehen. Polyvinylalkohol wird in der oben beschriebenen Weise vernetzt, um dem Angriff von Wasser bei höheren Temperaturen besser zu widerstehen.

Das erfindungsgemäße Verfahren zur Abtrennung von C₁-C₃-Alkanolen von weiteren organischen Verbindungen mit höherer C-Zahl in Gegenwart von Wasser bringt deutliche Vorteile gegenüber der bisher bekannten Trennung dieser Art ohne Mitverwendung von Wasser. So wird ein erhöhter Fluß des abzutrennenden Alkanols beobachtet, obwohl zu erwarten gewesen wäre, daß die hydrophile Membran in überwiegendem Maße nur das Wasser hindurchtreten ließe, während das Alkanol mit den weiteren organischen Verbindungen im Retentat verbleibt. Tatsächlich ist aber das Gegenteil der Fall, nämlich das hindurchtretende Wasser wirkt als eine Art Schleppmittel für das niedere Alkanol, so daß durch den erhöhten Fluß der spezifische Membranflächenbedarf stark reduziert werden kann. Setzt man beispielsweise den Membranflächenbedarf bei einer Pervaporation von Methanol aus i-Propanol als der weiteren organischen Verbindung mit 100 % an, so kann dieser Membranflächenbedarf auf 16 % des bisherigen Wertes gesenkt werden, wenn man etwa 8 bis 10 Gew.-% Wasser in einem Methanol/i-Propanol-Gemisch während der Pervaporation aufrechterhält.

### Beispiele

### Beispiel 1

Bei der Dampfpermeation von Methanol aus i-Propanol als der weiteren organischen Verbindung kann der Membranflächenbedarf auf 10 % gesenkt werden, wenn man dem Methanol/i-Propanol-Gemisch Wasser bis zu einem Gehalt von etwa 8 bis 10 Gew.-% Wasser zusetzt und den eingestellten Wassergehalt während der Dampfpermeation aufrechterhält.

### Beispiel 2

Bei der Dampfpermeation von Methanol aus Dimethylcarbonat als der weiteren organischen Verbindung wird die Permeation von Methanol erst ermöglicht, wenn man einem Methanol/Dimethylcarbonat-Gemisch Wasser bis zu einem Gehalt von etwa 8 bis 10 Gew.-% Wasser zusetzt und den eingestellten Wassergehalt während der Dampfpermeation aufrechterhält.

## Patentansprüche

1. Verfahren zur Abtrennung eines ersten Alkanols mit 1 bis 3 C-Atomen aus ihrem Gemisch mit weiteren organischen Verbindungen aus der Gruppe, die ein zweites geradkettiges oder verzweigtes, offenkettiges oder cyclisches, gesättigtes oder ungesättigtes C₂-C₁₀-Alkanol, einen C₂-C₈-Polyalkohol, einen geradkettigen oder verzweigten, offenkettigen oder cyclischen C₄-C₈-Ether oder -Polyether, eine geradkettige oder verzweigte, offenkettige oder cyclische C₂-C₆-Oxoverbindung, einen geradkettigen, cyclischen oder verzweigten C₂-C₉-Carbonsäureester, einen C₃-C₉-Kohlensäureester, einen Phosphorsäureester mit C₁-C₄-Alkylgruppen, einen 5-bis 7-gliedrigen aromatischen oder nicht aromatischen N-Heterocyclus, ein Sulfoxid oder Sulfon mit 2 bis 8 C-Atomen, einen C₁-C₄-Halogenaliphaten, ein C₃-C₈-Amin, ein C₃-C₈-Säureamid, einen C₅-C₈-Kohlenwasserstoff, eine geradkettige oder verzweigte C₂-C₆-Carbonsäure und ein C₂-C₆-Nitril umfaßt, wobei das erste Alkanol stets mindestens 1 C-Atom, vorzugsweise 2 C-Atome weniger hat als jede der weiteren organischen Verbindungen, wobei im Falle von Halogen-Aliphaten Halogensubstituenten als weitere C-Atome zählen, durch Pervaporation oder Dampfpermeation, dadurch gekennzeichnet, daß das beschriebene Gemisch unter Aufrechterhaltung eines Gehaltes an H₂O von 1 bis 30 Gew.-%, bevorzugt 5 bis 15 Gew.-%, bezogen auf die Menge von Gemisch und H₂O, bei 40 bis 130°C, bevorzugt bei 40 bis 100°C einer hydrophilen Membran zugeführt wird, wobei auf der Feed-Seite ein Druck von 0,5 bis 10 bar, bevorzugt 0,8 bis 6 bar, besonders bevorzugt 1 bis 5 bar, und auf der Permeatseite ein Druck von höchstens 100 mbar, bevorzugt höchstens 20 mbar, eingestellt wird und wobei als Permeat das erste Alkanol im Gemisch mit H₂O und als Retentat die weitere organische Verbindung in angereicherter Form erhalten werden und wobei nach Abtrennung des ersten Alkanols das vorhandene Wasser ebenfalls abgetrennt wird.

2. Verfahren nach Anspruch 1, dadurch gekennzeichnet, daß das erste Alkanol Ethanol oder Methanol oder ein Gemisch beider, bevorzugt Methanol ist.

3. Verfahren nach Anspruch 1, dadurch gekennzeichnet, daß das erste Alkanol von weiteren organischen Verbindungen aus der Gruppe der C₂-C₁₀-Alkohole, C₂-C₈-Polyalkohole, C₄-C₈-Ether, C₂-C₆-Oxoverbindungen, C₂-C₉-Carbonsäureester, C₃-C₉-Kohlensäureester, C₃-C₈-Amine, C₃-C₈-Säureamide und C₂-C₆-Carbonsäuren, bevorzugt von weiteren organischen Verbindungen aus der Gruppe der C₂-C₁₀-Alkohole, C₄-C₈-Ether, C₂-C₆-Oxoverbindungen, C₂-C₉-Carbonsäureester und C₃-C₉-Kohlensäureester abgetrennt wird.

4. Verfahren nach Anspruch 2, dadurch gekennzeichnet, daß Methanol aus seinem Gemisch mit einer weiteren Verbindung aus der Gruppe von geradkettigen oder verzweigten C₃-C₆-Alkanolen, Dimethylcarbonat, Methyl-tert.-butyl-ether oder tert.-Amyl-methyl-ether abgetrennt wird.

5. Verfahren nach Anspruch 1, dadurch gekennzeichnet, daß bei Durchführung als Pervaporation der Feed-Druck in Abhängigkeit von der Temperatur so eingestellt wird, daß er beim Siededruck oder oberhalb des Siededruckes des Feeds und bei Durchführung als Dampfpermeation der Feed-Druck in Abhängigkeit von der Temperatur so eingestellt wird, daß er unterhalb des Dampfdrucks des Feeds liegt.

6. Verfahren nach Anspruch 1, dadurch gekennzeichnet, daß der Gehalt an H₂O zu Beginn der Reaktion durch das im Gemisch ursprünglich vorhandene Wasser repräsentiert wird und im weiteren Verlauf Wasser in der Menge zugesetzt wird, die zur Aufrechterhaltung eines Gehaltes an H₂O von 1 bis 30 Gew.-%, bevorzugt 5 bis 15 Gew.-%, bezogen auf die Menge von Gemisch und Wasser, nötig ist.

7. Verfahren nach Anspruch 1, dadurch gekennzeichnet, daß eine Composit-Membran eingesetzt wird, die aus einem Trägermaterial und einer darauf angebrachten Schicht aus Cellulosediacetat, Cellulosetriacetat, Polyvinylalkohol oder durch Plasmapolymerisation aufgebrachten porenfreien Schicht besteht.

8. Verfahren nach Anspruch 7, dadurch gekennzeichnet, daß das Trägermaterial ein poröses, bevorzugt ein poröses asymmetrisches Polymer ist.

9. Verfahren nach Anspruch 7, dadurch gekennzeichnet, daß das Trägermaterial auf der Basis von Polyacrylnitril, Polysulfon, Polytetrafluorethylen, Polyethylen, Polyvinylalkohol, Polyethersulfon, Polyetherimid, Polyvinylidenfluorid, hydrolysiertem Cellulosetriacetat, Polyphenylensulfid oder Copolymeren von teilfluorierten Polyolefinen aufgebaut ist.

10. Verfahren nach Anspruch 7, dadurch gekennzeichnet, daß die aufgebrachte Schicht aus Polyvinylalkohol oder vernetztem Polyvinylalkohol besteht.

## Claims

1. Process for separating off a first alkanol having from 1 to 3 carbon atoms from its mixture with other organic compounds from the group comprising a second straight-chain or branched, open-chain or cyclic, saturated or unsaturated C₂-C₁₀-alkanol, a C₂-C₈-polyalcohol, a straight-chain or branched, open-chain or cyclic C₄-C₈-ether or polyether, a straight-chain or branched, open-chain or cyclic C₂-C₆-oxo compound, a straight-chain, cyclic or branched C₂-C₉-carboxylic ester, a C₃-C₉-carbonic ester, a phosphoric ester having C₁-C₄-alkyl groups, a from 5 to 7-membered aromatic or nonaromatic N-heterocycle, a sulphoxide or sulphone having from 2 to 8 carbon atoms, a C₁-C₄-halogenoaliphatic, a C₃-C₈-amine, a C₃-C₈-acid amide, a C₅-C₈-hydrocarbon, a straight-chain or branched C₂-C₆-carboxylic acid and a C₂-C₆-nitrile, with the first alkanol always having at least one carbon atom, preferably two carbon atoms, less than each of the other organic compounds, though in the case of halogenoaliphatics, halogen substituents are counted as further carbon atoms, by pervaporation or vapour permeation, characterized in that the mixture described is fed, while maintaining a water content from 1 to 30% by weight, preferably from 5 to 15% by weight, based on the amount of mixture and water, at from 40 to 130°C, preferably from 40 to 100°C, to a hydrophilic membrane, with a pressure from 0.5 to 10 bar, preferably from 0.8 to 6 bar, particularly preferably from 1 to 5 bar, being set on the feed side and a pressure of at most 100 mbar, preferably at most 20 mbar, being set on the permeate side and with the permeate obtained being the first alkanol in admixture with water and the retentate obtained being the other organic compound in enriched form and with, after separating off the first alkanol, the water present being likewise separated off.

2. Process according to Claim 1, characterized in that the first alkanol is ethanol or methanol or a mixture of the two, preferably methanol.

3. Process according to Claim 1, characterized in that the first alkanol is separated off from other organic compounds from the group comprising C₂-C₁₀-alcohols, C₂-C₈-polyalcohols, C₄-C₈-ethers, C₂-C₆-oxo compounds, C₂-C₉-carboxylic esters, C₃-C₉-carbonic esters, C₃-C₈-amines, C₃-C₈-acid amides and C₂-C₆-carboxylic acids, preferably from other organic compounds from the group comprising C₂-C₁₀-alcohols, C₄-C₈-ethers, C₂-C₆-oxo compounds, C₂-C₉-carboxylic esters and C₃-C₉-carbonic esters.

4. Process according to Claim 2, characterized in that methanol is separated off from a mixture thereof with one other compound from the group comprising straight-chain or branched C₃-C₆-alkanols, dimethyl carbonate, methyl tert-butyl ether or tert-amyl methyl ether.

5. Process according to Claim 1, characterized in that when carried out as pervaporation the feed pressure, as a function of the temperature, is set in such a way that it lies at the boiling pressure or above the boiling pressure of the feed and when carried out as vapour permeation the feed pressure, as a function of the temperature, is set in such a way that it lies below the vapour pressure of the feed.

6. Process according to Claim 1, characterized in that the water content at the beginning of the reaction is that amount of water originally present in the mixture and in the further course of the process water is added in the amount required to maintain a water content from 1 to 30% by weight, preferably from 5 to 15% by weight, based on the amount of mixture and water.

7. Process according to Claim 1, characterized in that the membrane used is a composite membrane comprising a support material and a layer of cellulose diacetate, cellulose triacetate or polyvinyl alcohol applied thereon or a pore-free layer applied by plasma polymerization.

8. Process according to Claim 7, characterized in that the support material is a porous polymer, preferably a porous asymmetric polymer.

9. Process according to Claim 7, characterized in that the support material is based on polyacrylonitrile, polysulphone, polytetrafluoroethylene, polyethylene, polyvinyl alcohol, polyethersulphone, polyetherimide, polyvinylidene fluoride, hydrolysed cellulose triacetate, polyphenylene sulphide or copolymers of partially fluorinated polyolefins.

10. Process according to Claim 7, characterized in that the layer applied comprises polyvinyl alcohol or crosslinked polyvinyl alcohol.

## Revendications

1. Procédé pour la séparation d'un premier alcanol contenant de 1 à 3 atomes de carbone de son mélange avec d'autres composés organiques choisis parmi le groupe qui comprend un second alcanol en C₂-C₁₀ à chaîne droite ou ramifiée, à chaîne ouverte ou cyclique, saturé ou insaturé, un polyalcool en C₂-C₈, un éther ou un polyéther en C₄-C₈ à chaîne droite ou ramifiée, à chaîne ouverte ou cyclique, un composé oxo en C₂-C₆ à chaîne droite ou ramifiée, à chaîne ouverte ou cyclique, un ester carboxylique en C₂-C₉ à chaîne droite, cyclique ou ramifiée, un ester d'acide carbonique en C₃-C₉, un ester phosphorique contenant des groupes alkyle en C₁-C₄, un composé hétérocyclique azoté penta- à heptagonal, aromatique ou non aromatique, un sulfoxyde ou une sulfone contenant de 2 à 8 atomes de carbone, un composé aliphatique halogéné en C₁-C₄, une amine en C₃-C₈, un amide d'acide en C₃-C₈, un hydrocarbure en C₅-C₈, un acide carboxylique en C₂-C₆ à chaîne droite ou ramifiée et un nitrile en C₂-C₆, dans lequel le premier alcanol contient toujours au moins 1 atome de carbone, de préférence 2 atomes de carbone de moins que chacun des autres composés organiques, dans lequel, dans le cas de composés aliphatiques halogénés, les substituants halogéno sont comptés comme atomes de carbone supplémentaires, par pervaporation ou par perméation à la vapeur, caractérisé en ce qu'on achemine à une membrane hydrophile le mélange décrit, tout en maintenant une teneur en H₂O de 1 à 30% en poids, de préférence de 5 à 15% en poids rapportés à la quantité du mélange et de H₂O, à une température de 40 à 130°C, de préférence à une température de 40 à 100°C, en réglant du côté alimentation une pression de 0,5 à 10 bar, de préférence de 0,8 à 6 bar, de manière particulièrement préférée de 1 à 5 bar, et du côté perméat, une pression maximale de 100 mbar, de préférence une pression maximale de 20 mbar, et dans lequel on obtient, à titre de perméat, le premier alcanol en mélange avec H₂O et, à titre de rétentat, l'autre composé organique sous forme enrichie, et dans lequel on sépare également l'eau présente, après la séparation du premier alcanol.

2. Procédé selon la revendication 1, caractérisé en ce que le premier alcanol représente l'éthanol, le méthanol ou encore un mélange des deux, de préférence le méthanol.

3. Procédé selon la revendication 1, caractérisé en ce qu'on sépare le premier alcanol d'autres composés organiques choisis parmi le groupe comprenant des alcools en C₂-C₁₀, des polyalcools en C₂-C₈, des éthers en C₄-C₈, des composés oxo en C₂-C₆, des esters carboxyliques en C₂-C₉, des esters d'acide carbonique en C₃-C₉, des amines en C₃-C₈, des amides d'acides en C₃-C₈ et des acides carboxyliques en C₂-C₆, de préférence d'autres composés organiques choisis parmi le groupe comprenant des alcools en C₂-C₁₀, des éthers en C₄-C₈, des composés oxo en C₂-C₆, des esters carboxyliques en C₂-C₉ et des esters d'aide carbonique en C₃-C₉.

4. Procédé selon la revendication 2, caractérisé en ce qu'on sépare le méthanol de son mélange avec un autre composé choisi parmi le groupe comprenant des alcanols en C₃-C₆ à chaîne droite ou ramifiée, le diméthylcarbonate, l'éther méthyl-tert.-butylique ou l'éther tert.-amyl-méthylique.

5. Procédé selon la revendication 1, caractérisé en ce que, dans le mode opératoire par pervaporation, on règle la pression d'alimentation en fonction de la température de telle sorte qu'elle se situe au niveau de la pression d'ébullition ou au-delà de la pression d'ébullition de l'alimentation et, dans le mode opératoire par perméation de la vapeur, on règle la pression d'alimentation en fonction de la température de telle sorte qu'elle se situe en dessous de la pression de vapeur de l'alimentation.

6. Procédé selon la revendication 1, caractérisé en ce que la teneur en H₂O au début de la réaction est représentée par l'eau présente à l'origine dans le mélange et, dans la suite du processus, on ajoute la quantité d'eau requise pour maintenir une teneur en H₂O de 1 à 30% en poids, de préférence de 5 à 15% en poids rapportés à la quantité du mélange et de l'eau.

7. Procédé selon la revendication 1, caractérisé en ce qu'on met en oeuvre une membrane composite qui est constituée par une matière de support et par une couche appliquée sur cette dernière, constituée par du diacétate de cellulose, du triacétate de cellulose, de l'alcool polyvinylique, ou par une couche exempte de pores appliquée par polymérisation au plasma.

8. Procédé selon la revendication 7, caractérisé en ce que la matière de support est un polymère poreux, de préférence un polymère asymétrique poreux.

9. Procédé selon la revendication 7, caractérisé en ce que la matière de support est réalisée à base de polyacrylonitrile, de polysulfone, de polytétrafluoréthylène, de polyéthylène, d'alcool polyvinylique, de polyéther-sulfone, de polyétherimide, de fluorure de polyvinylidène, de triacétate de cellulose hydrolysé, de polyphénylènesulfure ou encore de copolymères de polyoléfines partiellement fluorées.

10. Procédé selon la revendication 7, caractérisé en ce que la couche appliquée est constituée d'alcool polyvinylique ou d'alcool polyvinylique réticulé.
